(19) Europäisches Patentamt
European Patent Office
Office européen des brevets

(11) EP 3 811 925 A1

(12) EUROPEAN PATENT APPLICATION
published in accordance with Art. 153(4) EPC

(43) Date of publication:
**28.04.2021 Bulletin 2021/17**

(21) Application number: **19819321.1**

(22) Date of filing: **10.06.2019**

(51) Int Cl.:
***A61K 8/898*** *(2006.01)* ***A61K 8/362*** *(2006.01)*
***A61K 8/365*** *(2006.01)* ***A61K 8/39*** *(2006.01)*
***A61K 8/46*** *(2006.01)* ***A61Q 5/02*** *(2006.01)*
***C11D 1/37*** *(2006.01)* ***C11D 1/83*** *(2006.01)*
***C11D 1/94*** *(2006.01)* ***C11D 3/20*** *(2006.01)*
***C11D 3/37*** *(2006.01)*

(86) International application number:
**PCT/JP2019/022962**

(87) International publication number:
**WO 2019/240088 (19.12.2019 Gazette 2019/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.06.2018 JP 2018112237**

(71) Applicant: **Kao Corporation**
**Chuo-ku,**
**Tokyo 103-8210 (JP)**

(72) Inventor: **YAMAZAKI, Naoyuki**
**Tokyo 131-8501 (JP)**

(74) Representative: **Hoffmann Eitle**
**Patent- und Rechtsanwälte PartmbB**
**Arabellastraße 30**
**81925 München (DE)**

# (54) DETERGENT

(57) The present invention relates to a detergent containing or prepared by blending: (A) an anionic surfactant; (B) an aminopolyether-modified silicone containing a polyoxyalkylene structure and satisfying the following formula (I); and (C) an organic acid, wherein a mass ratio [(A)/(B)] of the component (A) to the component (B) is less than 25, and a mass ratio [(A)/(C)] of the component (A) to the component (C) is 30 or more and 3,000 or less:

$$0.01 \le \mathrm{Si/AO} \le 1.6 \qquad \text{(I)}$$

(in the formula (I), Si represents a molar number of a silicon atom in the component (B), and AO represents an oxyalkylene average addition molar number in the component (B)).

EP 3 811 925 A1

## Description

Field of the Invention

The present invention relates to a detergent.

Background of the Invention

There is a demand for a hair cosmetic capable of reducing the time and effort in daily hair-care activities and leading a hair-care life without stress. In particular, in the field of hair detergents, in addition to the basic function of removing hair stains, research has been conducted on hair detergents having a good feeling of use, such as good lathering, and putting fingers through hair during washing and after rinsing.
For example, PTL 1 discloses that a hair cosmetic containing a reactive silicone-based block copolymer has good lathering and emulsifying properties, and when treating hair, has excellent adhesive effect of split ends, feel, and antistatic properties.
PTL 2 discloses that a hair cosmetic containing a reactive silicone-based block copolymer, cationic galactomannan, and an N-acyl-N-methyltaurine type anionic surfactant is excellent in lathering and finished velvet feeling.

Citation List

Patent Literature

PTL 1: JP 9-151119 A
PTL 2: JP 2007-161597 A

Summary of the Invention

The present invention relates to the following.
A detergent contains or prepared by blending:

(A) an anionic surfactant,
(B) an aminopolyether-modified silicone containing a polyoxyalkylene structure and satisfying the following formula (I):

$$0.01 \leq \mathrm{Si/AO} \leq 1.6 \qquad \text{(I)}$$

wherein Si represents a molar number of a silicon atom in the component (B), and AO represents an oxyalkylene average addition molar number in the component (B), and
(C) an organic acid,
wherein a mass ratio [(A)/(B)] of the component (A) to the component (B) is less than 25, and a mass ratio [(A)/(C)] of the component (A) to the component (C) is 30 or more and 3,000 or less.

Detailed Description of the Invention

[Detergent]

The detergent of the present invention is one containing or one prepared by blending: (A) an anionic surfactant, (B) an aminopolyether-modified silicone containing a polyoxyalkylene structure and satisfying the following formula (I):

$$0.01 \leq \mathrm{Si/AO} \leq 1.6 \qquad \text{(I)}$$

(in the formula (I), Si represents a molar number of a silicon atom in the component (B), and AO represents an oxyalkylene average addition molar number in the component (B)), and (C) an organic acid, wherein a mass ratio [(A)/(B)] of the

component (A) to the component (B) is less than 25, and a mass ratio [(A)/(C)] of the component (A) to the component (C) is 30 or more and 3,000 or less.

In view of the fact that the detergent of the present invention has the aforementioned constitution, it is favorable in lathering during washing and is able to effectively inhibit and solve the generation of entanglement of a washing object, such as hair and fibers, during washing and even after washing. For example, in the case where the detergent of the present invention is a hair detergent, only by washing the hair with the foregoing hair detergent, the entanglement of hair can be inhibited, and even when an operation of applying a force from the outside to dissociate the hair is not performed, the entanglement of hair can be spontaneously dissociated. The expression "one containing the components (A), (B), and (C)" also means "one prepared by blending the components (A), (B), and (C)".

The hair is damaged by the living environment (ultraviolet rays or heat of sunlight), the daily hair-care activities (friction by hair washing or brushing), and the chemical treatment (coloring, perm, etc.). When the damaged hairs rub against each other, a large frictional force is generated on the surface, and entanglement is caused. The "entanglement of hair" is a cause of all of stresses in the hair-care activities, and there was involved such a problem that when the entanglement of hair is once generated, it is difficult to dissociate the entanglement. Although the entanglement of hair is possibly generated during hair washing, during conditioning, after towel drying, or during drying with a hair dryer or the like, according to the research made by the present inventor, it has been noted that when the entanglement of hair is generated at the stage of hair washing, even if a conditioning treatment is subsequently performed, it is difficult to completely dissociate the entanglement. Accordingly, it is preferred that the entanglement of hair can be inhibited or solved during hair washing.

In particular, when performing an action to allow hairs to rub against each other by, for example, towel drying, the hairs which have been dissociated through the conditioning treatment are again strongly entangled in cooperation with friction with the towel. Accordingly, massive time and effect are taken for the action to dissociate the entanglement in addition to drying, and a long time is required for drying.

PTLs 1 and 2 do not disclose inhibition and solution of the entanglement of hair on the occasion of hair washing, in particular, spontaneous dissociation of the entanglement of hair without performing an operation of putting fingers through hair or the like.

The present invention is concerned with a detergent that is favorable in lathering during washing and is able to inhibit and solve the generation of entanglement of a washing object, such as hair and fibers, even during washing and after washing without performing an operation of putting fingers through hair or the like.

The present inventor has found that the aforementioned problem can be solved by a detergent containing or prepared by blending: an anionic surfactant, an aminopolyether-modified silicone containing a polyoxyalkylene structure and satisfying a predetermined requirement, and an organic acid in predetermined proportions, respectively.

In accordance with the detergent of the present invention, lathering during washing is favorable, and the generation of entanglement of a washing object, such as hair and fibers, even during washing and after washing can be substantially inhibited and solved without performing an operation of putting fingers through hair or the like. For example, in the case where the detergent of the present invention is a hair detergent, the entanglement in hair after washing and towel drying is hardly generated, and therefore, the hair can be dried for a short time, and finish after drying becomes favorable.

Although the reason why in view of the fact that the detergent of the present invention has the aforementioned constitution, the effects of the present invention are brought is not elucidated yet, the following may be conjectured.

In order to reveal such a state that the entanglement of hair or the like is spontaneously dissociated during washing and after washing of hair or fibers, it may be considered to be important to lower a frictional force working on the hair or between the fibers. In a hair detergent, a silicone is generally blended as an active ingredient; however, the present inventor has been found that in order to lower a frictional force working on the hair or between the fibers, it is effective to not only use the component (B) that is a water-soluble silicone satisfying a predetermined requirement but also blend the component (B) in a predetermined proportion relative to the component (A).

In addition, though the anionic surfactant that is the component (A) is an essential component for imparting detergency, since the component (B) is cationic, in the case where the components (A) and (B) are coexistent, charges are neutralized due to an electrostatic interaction, to cause insolubilization, so that the function to remove the entanglement cannot be exhibited. Accordingly, in the detergent of the present invention, the organic acid that is the component (C) is used as a component for inhibiting the insolubilization to be caused due to the interaction between the components (A) and (B). Since the component (C) is stronger in the electrostatic interaction with the cationic component (B) than the component (A), it may be considered that by blending the component (C) in a predetermined proportion relative to the component (A), the interaction between the components (A) and (B) in the detergent can be effectively inhibited, and the component (C) can effectively act on the hair or between the fibers.

When towel drying is performed in a state that the entanglement of hair is dissociated, a contact area between the hair and the towel increases so that the moisture of the hair is quickly absorbed onto the towel. In addition, it may be considered that a contact area between the hair and air increases during drying with a hair dryer or during natural

drying, and the moisture in the hair is quickly transpired, so that a drying speed of the hair is improved.

From the viewpoint of effectively obtaining the effects of the present invention, the detergent of the present invention is preferably a hair detergent or a detergent for fibers, and more preferably a hair detergent.

A dosage form of the detergent is not particularly limited, and it is possible to adopt an arbitrary dosage form, for example, a liquid form, a lathering form, a paste form, a cream form, a solid form, or a powder form. For example, in the case of a hair detergent, a liquid form, a paste form or a cream form is preferred, and a liquid form is more preferred.

<Component (A): Anionic Surfactant>

The detergent of the present invention contains or is prepared by blending therein the anionic surfactant as the component (A). The component (A) is used for the purpose of imparting detergency.

Examples of the anionic surfactant include alkylbenzenesulfonic acid salts, alkyl or alkenyl ether sulfuric acid salts, alkyl or alkenyl sulfuric acid salts, alkylsulfonic acid salts, saturated or unsaturated fatty acid salts, alkyl or alkenyl ether carboxylic acid salts, $\alpha$-sulfo fatty acid salts, N-acylamino acids, phosphoric acid mono- or diesters, and sulfosuccinic acid esters. Of these, one or more thereof may be used.

Examples of a counter ion of the anionic group of the anionic surfactant include alkali metal ions, such as a sodium ion and a potassium ion; alkaline earth metal ions, such as a calcium ion and a magnesium ion; an ammonium ion; and alkanol ammoniums having 1 to 3 alkanol groups having 2 or 3 carbon atoms (for example, monoethanolammonium, diethanolammonium, triethanolammonium, and triisopropanolammonium).

Above all, from the viewpoint of lathering during washing, the component (A) is preferably at least one selected from the group consisting of alkyl sulfuric acid salts, alkyl ether sulfuric acid salts, and alkyl ether carboxylic acid salts. Examples of the alkyl ether sulfuric acid salt include polyoxyethylene alkyl ether sulfuric acid salts, such as ammonium laureth sulfate; and examples of the alkyl ether carboxylic acid salts include polyoxyethylene alkyl ether acetic acid salts, such as sodium laureth acetate.

From the viewpoint of lathering during washing, the compound (A) is more preferably at least one selected from the group consisting of alkyl ether sulfuric acid salts and alkyl ether carboxylic acid salts; still more preferably at least one selected from the group consisting of polyoxyethylene alkyl ether sulfuric acid salts and polyoxyethylene alkyl ether acetic acid salts; and yet still more preferably at least one selected from the group consisting of ammonium laureth sulfate and sodium laureth acetate.

From the viewpoint of lathering during washing and inhibition and solution of entanglement of a washing object, such as hair, and the viewpoint of reduction in entanglement during hair rinsing, easiness of brushing after towel drying, and quick drying properties in the hair detergent, the content or blending amount of the component (A) in the detergent is preferably 1% by mass or more, more preferably 3% by mass or more, and still more preferably 5% by mass or more, and from the same viewpoints, it is preferably 30% by mass or less, more preferably 20% by mass or less, and still more preferably 18% by mass or less. A specific range of the content or blending amount of the component (A) in the detergent is preferably 1 to 30% by mass, more preferably 3 to 20% by mass, and still more preferably 5 to 18% by mass.

<Component (B): Aminopolyether-modified Silicone Containing a Polyoxyalkylene Structure>

The detergent of the present invention contains or is prepared by blending therein: an aminopolyether-modified silicone containing a polyoxyalkylene structure and satisfying the following formula (I) as the component (B):

$$0.01 \leq \mathrm{Si/AO} \leq 1.6 \qquad \text{(I)}$$

(in the formula (I), Si represents a molar number of a silicon atom in the component (B), and AO represents an oxyalkylene average addition molar number in the component (B)).

In view of the fact that the detergent of the present invention contains or is prepared by blending therein: the component (B), in the washing object, such as hair and fibers, the generation of entanglement can be effectively inhibited and solved owing to the aforementioned mechanism of action even during washing and after washing.

From the viewpoint of inhibition and solution of entanglement of a washing object, such as hair, and reduction in entanglement during hair rinsing, easiness of brushing after towel drying, and quick drying properties in the hair detergent, the Si/AO in the formula (I) is 0.01 or more, preferably 0.05 or more, still more preferably 0.1 or more, yet still more preferably 0.3 or more, even yet still more preferably 0.5 or more, and even still more preferably 0.7 or more, and from the same viewpoint, it is 1.6 or less, preferably 1.4 or less, more preferably 1.2 or less, still more preferably 1.0 or less, and yet still more preferably 0.9 or less. When the Si/AO of the component (B) is 0.01 or more and 1.6 or less, the effect for inhibiting and solving the entanglement of a washing object, such as hair, the easiness of brushing after towel

drying, and the quick drying properties are favorable.

The Si/AO in the formula (I) can be calculated from an integrated value of hydrogen atoms bonded to silicon and H of the hydrocarbon group and an integrated value of H of the oxyalkylene group, as measured by the $^1$H-NMR measurement. Specifically, the measurement may be performed by the method described in the section of Examples.

From the viewpoint of lathering during washing, inhibition and solution of entanglement of a washing object, such as hair, and reduction in entanglement during hair rinsing, easiness of brushing after towel drying, and quick drying properties in the hair detergent, the nitrogen content of the component (B) is preferably 0.1% by mass or more, more preferably 0.2% by mass or more, still more preferably 0.5% by mass or more, and yet still more preferably 1.0% by mass or more, and it is preferably 2.5% by mass or less, more preferably 2.0% by mass or less, still more preferably 1.8% by mass or less, and yet still more preferably 1.5% by mass or less. A specific range of the nitrogen content of the component (B) is preferably 0.1 to 2.5% by mass, more preferably 0.2 to 2.0% by mass, still more preferably 0.5 to 1.8% by mass, and yet still more preferably 1.0 to 1.5% by mass. The nitrogen content of the component (B) is a value measured in conformity with the potentiometric titration method as prescribed in JIS K0113:2005.

More specifically, the component (B) is preferably an aminopolyether-modified silicone having a repeating unit represented by the following general formula (1), and more preferably an aminopolyether- modified silicone constituted of a repeating unit represented by the following general formula (1).

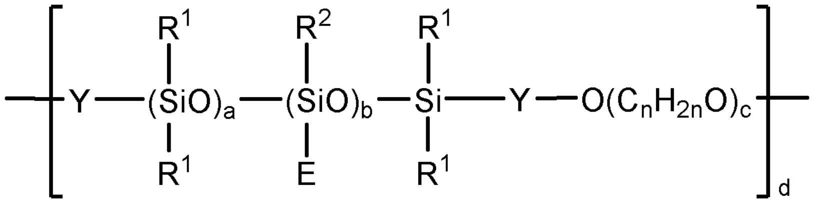

(1)

In the formula (1), $R^1$ represents a hydrogen atom or a monovalent hydrocarbon group having 1 or more and 6 or less carbon atoms. $R^2$ represents either $R^1$ or E. E represents a monovalent group represented by $-R^3-Z$ (wherein $R^3$ represents a single bond or a divalent hydrocarbon group having 1 or more and 20 or less carbon atoms; and Z represents a primary to tertiary amino group-containing group). Y represents an alkylene group having 1 or more and 6 or less carbon atoms. a represents a number of 2 or more; b represents a number of 1 or more; c represents a number of 4 or more and 100 or less; and d represents a number of 1 or more. n represents a number of 2 or more and 10 or less. The bonding order among the structural units within the bracket does not matter, and the bonding form may be a block form or a random form. The $C_nH_{2n}O$'s in the number of c may be the same as or different from each other. In addition, plural $R^1$'s, $R^2$'s, and E's may be the same as or different from each other, respectively.

In the case where the component (B) is constituted of the repeating unit represented by the general formula (1), the Si/AO in the foregoing formula (I) is represented by (a + b + 1)/c.

In the general formula (1), $R^1$ represents a hydrogen atom or a monovalent hydrocarbon group having 1 or more and 6 or less carbon atoms. $R^1$'s are each independently preferably a hydrogen atom, an alkyl group having 1 or more and 6 or less carbon atoms, or a phenyl group, more preferably a methyl group or an ethyl group, and still more preferably a methyl group.

In the general formula (1), $R^2$ represents either $R^1$ or E. E represents a monovalent group represented by $-R^3-Z$ (wherein $R^3$ represents a single bond or a divalent hydrocarbon group having 1 or more and 20 or less carbon atoms).

$R^3$ is preferably a divalent hydrocarbon group having 1 or more and 20 or less carbon atoms; more preferably an alkylene group having 1 or more and 20 or less carbon atoms; still more preferably a linear or branched alkylene group having 1 or more and 6 or less carbon atoms; yet still more preferably a methylene group, an ethylene group, a trimethylene group, a propylene group, a tetramethylene group, or a hexamethylene group; and even yet still more preferably a trimethylene group or a propylene group.

Z is a primary to tertiary amino group-containing group, and preferably an amino group-containing group represented by $-N(R^4)_2$, $-NR^4(CH_2)_mN(R^4)_2$, or $-NR^4(CH_2)_mN(R^5)CO-R^6$. Here, $R^4$ and $R^5$ each independently represent a hydrogen atom or an alkyl group having 1 or more and 3 or less carbon atoms, and preferably a hydrogen group or a methyl group. $R^6$ represents an alkyl group having 1 or more and 3 or less carbon atoms. m represents a number of 1 or more and 6 or less, and preferably a number of 2 or more and 4 or less.

In the general formula (1), the E group is preferably $-(CH_2)_3-NH_2$, $-(CH_2)_3-N(CH_3)_2$, $-(CH_2)_3-NH-(CH_2)_2-NH_2$, or $-(CH_2)_2-NH-(CH_2)_2-N(CH_3)_2$, and more preferably $-(CH_2)_3-NH-(CH_2)_2-NH_2$.

In the general formula (1), Y is an alkylene group having 1 or more and 6 or less carbon atoms, preferably an ethylene group, a propylene group, a trimethylene group, a n-butylene group (tetramethylene group), or an i-butylene group, and more preferably a n-butylene group or an i-butylene group. The i-butylene group as referred to herein includes $-CH(CH_3)CH_2CH_2-$, $-CH_2CH(CH_3)CH_2-$, and $-CH_2CH_2CH(CH_3)-$.

In the general formula (1), a represents a number of 2 or more; b represents a number of 1 or more; c represents a number of 4 or more and 100 or less; and d represents a number of 1 or more. a is preferably a number of 2 or more and 1,000 or less, and more preferably a number of 2 or more and 100 or less. b is preferably a number of 1 or more and 50 or less; c is preferably a number of 4 or more and 50 or less, and more preferably a number of 10 or more and 18 or less; and d is preferably a number of 1 or more and 100 or less.

In the general formula (1), n represents a number of 2 or more and 10 or less, preferably a number of 2 or more and 6 or less, and more preferably a number of 2 or more and 4 or less.

The component (B) is more preferably an aminopolyether-modified silicone having a repeating unit represented by the following general formula (2), and still more preferably an aminopolyether-modified silicone constituted of a repeating unit represented by the following general formula (2). Even in the case where the component (B) is constituted of a repeating unit represented by the general formula (2), the Si/AO in the aforementioned formula (I) is represented by (a + b + 1)/c.

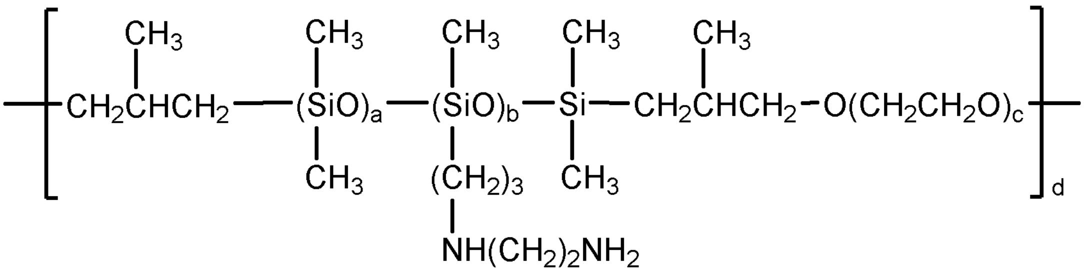 (2)

In the formula (2), a to d are the same as those mentioned above.

Although the component (B) may be any form of an oil, an emulsion, and so on, it is preferably an oil.

In the case where the component (B) is an oil, from the viewpoint of allowing it to efficiently remain in the hair or the like to exhibit the effects of the present invention, its kinematic viscosity at 25°C is preferably 50 $mm^2/s$ or more, more preferably 100 $mm^2/s$ or more, still more preferably 300 $mm^2/s$ or more, yet still more preferably 500 $mm^2/s$ or more, and even yet still more preferably 700 $mm^2/s$ or more, and from the viewpoint of water solubility, it is preferably 50,000 $mm^2/s$ or less, more preferably 20,000 $mm^2/s$ or less, still more preferably 10,000 $mm^2/s$ or less, yet still more preferably 5,000 $mm^2/s$ or less, even yet still more preferably 3,000 $mm^2/s$ or less, even still more preferably 1,500 $mm^2/s$ or less, and even still more further preferably 1,400 $mm^2/s$ or less. A specific range of the kinematic viscosity at 25°C of the component (B) is preferably 50 to 50,000 $mm^2/s$, more preferably 100 to 20,000 $mm^2/s$, still more preferably 300 to 10,000 $mm^2/s$, yet still more preferably 500 to 5,000 $mm^2/s$, even yet still more preferably 500 to 3,000 $mm^2/s$, even still more preferably 500 to 1,500 $mm^2/s$, and even still more further preferably 700 to 1,400 $mm^2/s$.

The kinematic viscosity at 25°C of the component (B) is a value measured in conformity with the "Viscosity Measurement Methods for Liquids" as prescribed in JIS Z8803:2011 or ASTM D445-46T, and may be, for example, measured with an Ubbelohde viscometer.

The component (B) may be used alone or in combination of two or more thereof.

As the component (B), a commercially available aminopolyether-modified silicone may be used, too. Examples of the aminopolyether-modified silicone having a repeating unit represented by the general formula (2) include "SILSTYLE 201", manufactured by Dow Corning Toray Co., Ltd. ((bisisobutyl PEG-14/amodimethicone) copolymer, nitrogen content: 1.2% by mass, viscosity: 1,000 $mm^2/s$, Si/AO = 0.80).

In the detergent of the present invention, from the viewpoint of lathering during washing and inhibition and solution of entanglement of a washing object, such as hair, and the viewpoint of reduction in entanglement during hair rinsing, easiness of brushing after towel drying, quick drying properties, and easiness of blending in the hair detergent, a mass ratio [(A)/(B)] of the component (A) to the component (B) is less than 25, preferably 24 or less, more preferably 22 or less, and still more preferably 20 or less, and it is preferably 0.1 or more, more preferably 0.5 or more, still more preferably 1 or more, yet still more preferably 3 or more, and even yet still more preferably 5 or more. A specific range pf the mass ratio [(A)/(B)] of the component (A) to the composition (B) in the detergent is less than 25, and it is preferably 0.1 to 24, more preferably 0.5 to 22, still more preferably 1 to 20, yet still more preferably 3 to 20, and even yet still more preferably 5 to 20.

Although the content or blending amount of the component (B) in the detergent may be the amount at which the mass ratio [(A)/(B)] is less than 25, from the viewpoint of inhibition and solution of entanglement of a washing object, such as hair, and the viewpoint of reduction in entanglement during hair rinsing, easiness of brushing after towel drying, and quick drying properties in the hair detergent, it is preferably 0.01% by mass or more, more preferably 0.05% by mass or more, and still more preferably 0.1% by mass or more, and from the viewpoint of lathering during washing, it is preferably 10% by mass or less, more preferably 5% by mass or less, and still more preferably 3% by mass or less. A

specific range of the content or blending amount of the component (B) in the detergent is preferably 0.01 to 10% by mass, more preferably 0.05 to 5% by mass, and still more preferably 0.1 to 3% by mass.

<Component (C): Organic Acid>

The detergent of the present invention contains or is prepared by blending therein an organic acid as the component (C). In view of the fact that the detergent of the present invention contains or is prepared by blending therein the component (C), the insolubilization to be caused due to the electrostatic interaction between the components (A) and (B) is inhibited, so that the function of the component (B) relative to a washing object, such as hair, can be effectively exhibited.

Specific examples of the organic acid include monovalent carboxylic acids, such as acetic acid, propionic acid, lactic acid, glycolic acid, gluconic acid, and benzoic acid; and polyvalent carboxylic acids, such as oxalic acid, malonic acid, succinic acid, adipic acid, fumaric acid, maleic acid, itaconic acid, citric acid, tartaric acid, malic acid, and phthalic acid; and besides, methanesulfonic acid, benzenesulfonic acid, and p-toluenesulfonic acid.

From the viewpoint of effectively exhibiting the function of the component (B) and more effectively inhibiting and solving the entanglement of a washing object, such as hair, the component (C) is preferably at least one selected from the group consisting of a monovalent carboxylic acid having a hydroxy group and a polyvalent carboxylic acid which may have a hydroxy group, and more preferably a polyvalent carboxylic acid which may have a hydroxy group.

More specifically, the component (C) is preferably at least one selected from the group consisting of lactic acid, succinic acid, citric acid, tartaric acid, and malic acid, more preferably at least one selected from the group consisting of lactic acid, succinic acid, citric acid, and malic acid, and still more preferably at least one selected from the group consisting of succinic acid, citric acid, and malic acid.

In the detergent of the present invention, from the viewpoint of inhibiting the insolubilization to be caused due to the electrostatic interaction between the component (A) and the component (B) to reveal the function of the component (B) and the viewpoint of reduction in entanglement during hair rinsing, easiness of brushing after towel drying, and quick drying properties and the viewpoint of easiness of blending in the hair detergent, a mass ratio [(A)/(C)] of the component (A) to the component (C) is 30 or more and 3,000 or less, and it is preferably 2,000 or less, still more preferably 1,000 or less, yet still more preferably 500 or less, and even yet still more preferably 200 or less, still more preferably 100 or less, and still more preferably 80 or less.

A specific range of the mass ratio [(A)/(C)] of the component (A) to the component (C) in the detergent is 30 to 3,000, preferably 30 to 2,000, more preferably 30 to 1,000, still more preferably 30 to 500, yet still more preferably 30 to 200, even yet still more preferably 30 to 100, and even still more preferably 30 to 80.

Although the content or blending amount of the component (C) in the detergent may be the amount at which the mass ratio [(A)/(C)] is 30 or more and 3,000 or less, from the viewpoint of inhibition and solution of entanglement of a washing object, such as hair, and the viewpoint of reduction in entanglement during hair rinsing, easiness of brushing after towel drying, and quick drying properties in the hair detergent, it is preferably 0.005% by mass or more, more preferably 0.01% by mass or more, still more preferably 0.05% by mass or more, yet still more preferably 0.1% by mass or more, and even yet still more preferably 0.2% or more. In addition, from the viewpoint of lathering during washing and inhibition and solution of entanglement of a washing object, such as hair, and the viewpoint of reduction in entanglement during hair rinsing, easiness of brushing after towel drying, quick drying properties, and easiness of blending in the hair detergent, the content or blending amount of the component (C) in the detergent is preferably 10% by mass or less, more preferably 5% by mass or less, still more preferably 3% by mass or less, and yet still more preferably 2% by mass or less, even yet still more preferably 1% by mass or less, and even still more preferably 0.5% by mass or less..

A specific range of the content or blending amount of the component (C) in the detergent is preferably 0.005 to 10% by mass, more preferably 0.01 to 5% by mass, still more preferably 0.05 to 3% by mass, and yet still more preferably 0.1 to 2% by mass, even yet still more preferably 0.1 to 1% by mass, even still more preferably 0.1 to 0.5% by mass, and even still more further preferably 0.2 to 0.5% by mass.

<Component (B)': Silicone Other Than Component (B)>

From the viewpoint of reducing the entanglement during hair rinsing in the hair detergent, the detergent of the present invention may be one further containing or being prepared by further blending, as a component (B)', a silicone other than the component (B). It is preferred that the component (B)' is water-insoluble. In this specification, the expression "water-insoluble" means that the solubility in 100 g of water at 25°C is 0.1 g or less.

Examples of the component (B)' include at least one selected from the group consisting of an aminopolyether-modified silicone other than the component (B), an amino-modified silicone, a polyether-modified silicone, a dimethylpolysiloxane (dimethicone), a methylphenylpolysiloxane, a fatty acid-modified silicone, an alkoxy-modified silicone, and an alkyl-modified silicone. Of these, at least one selected from the group consisting of an aminopolyether-modified

silicone other than the component (B) and a dimethylpolysiloxane is preferred.

The aminopolyether-modified silicone other than the component (B) is preferably an aminopolyether-modified silicone (B1)' (hereinafter simply referred to as "component (B1)") containing a polyoxyalkylene structure and satisfying the following formula (II):

$$1.6 < Si/AO \leq 3.0 \qquad \text{(II)}$$

In the formula (II), Si represents a molar number of a silicon atom in the component (B1)', and AO represents an oxyalkylene average addition molar number in the component (B1)'.

In the formula (II), from the viewpoint of water-insolubility, the Si/AO is preferably 1.8 or more, more preferably 2.0 or more, and still more preferably 2.2 or more, and from the viewpoint of blending stability, it is preferably 2.8 or less, and more preferably 2.6 or less. The Si/AO may be determined by the same method as that mentioned above.

From the viewpoint of lathering during washing and reduction in entanglement during hair rinsing, easiness of brushing after towel drying, and quick drying properties in the hair detergent, the nitrogen content of the component (B1)' is preferably 0.1% by mass or more, more preferably 0.2% by mass or more, still more preferably 0.5% by mass or more, and yet still more preferably 0.8% by mass or more, and it is preferably 2.5% by mass or less, more preferably 2.0% by mass or less, still more preferably 1.8% by mass or less, and yet still more preferably 1.5% by mass or less. A specific range of the nitrogen content of the component (B1)' is preferably 0.1 to 2.5% by mass, more preferably 0.2 to 2.0% by mass, still more preferably 0.5 to 1.8% by mass, and yet still more preferably 0.8 to 1.5% by mass. The nitrogen content may be determined by the same method as that mentioned above.

It is more preferred that the component (B1)' has a repeating unit represented by the following general formula (1). More preferably, the component (B1)' is an aminopolyether-modified silicone constituted of a repeating unit represented by the following general formula (1).

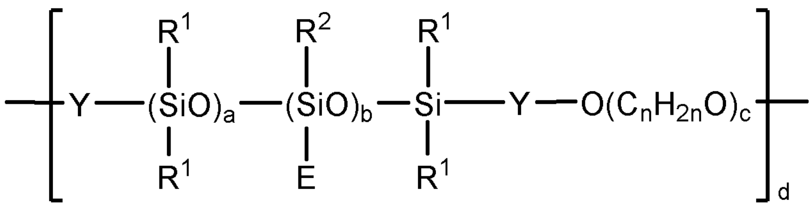

(1)

In the formula (1), $R^1$, $R^2$, E, Y, a to d, and n are the same as those mentioned above.

In the case where the component (B1)' is constituted of the repeating unit represented by the general formula (1), the Si/AO in the formula (II) is represented by $(a + b + 1)/c$.

In the component (B1)', $R^1$, $R^2$, E, Y, a to d, and n in the formula (1) and preferred ranges thereof are the same as those in the component (B) except for the range of Si/AO. That is, the component (B1)' is more preferably an aminopolyether-modified silicone having the repeating unit represented by the foregoing general formula (2), and sill more preferably an aminopolyether-modified silicone constituted of the repeating unit represented by the foregoing general formula (2).

Although the component (B1)' may be any form of an oil, an emulsion, and so on, it is preferably an oil.

In the case where the component (B1)' is an oil, from the viewpoint of water-insolubility, its kinematic viscosity at 25°C is preferably 2,000 $mm^2/s$ or more, more preferably 3,000 $mm^2/s$ or more, and still more preferably 5,000 $mm^2/s$ or more, and from the viewpoint of easiness of blending, it is preferably 200,000 $mm^2/s$ or less, more preferably 100,000 $mm^2/s$ or less, and still more preferably 50,000 $mm^2/s$ or less. In the case where the component (B1)' is an oil, a specific range of the kinematic viscosity at 25°C is preferably 2,000 to 200,000 $mm^2/s$, more preferably 3,000 to 100,000 $mm^2/s$, and still more preferably 5,000 to 50,000 $mm^2/s$. The kinematic viscosity may be measured by the same method as that mentioned above.

As the component (B1)', a commercially available aminopolyether-modified silicone may be used, too. Examples thereof include "SILSTYLE 104", manufactured by Dow Corning Toray Co., Ltd. ((bisisobutyl PEG-14/amodimethicone) copolymer having the repeating unit represented by the general formula (2), nitrogen content: 1.2% by mass, kinematic viscosity at 25°C: 12,000 $mm^2/s$, Si/AO = 2.46); "SS-3588", manufactured by Dow Corning Toray Co., Ltd. ((bisisobutyl PEG-15/amodimethicone) copolymer having the repeating unit represented by the foregoing general formula (2), nitrogen content: 1.0% by mass, Si/AO = 2.39); and "SILSTYLE 401", manufactured by Dow Corning Toray Co., Ltd. ((bisisobutyl PEG/PPG-20/35/amodimethicone) copolymer, nitrogen content: 0.2% by mass).

The dimethylpolysiloxane as the component (B)' may be any form of an oil, an emulsion, a solution prepared

by diluting a highly polymerized dimethylpolysiloxane with a low-viscosity silicone or a liquid paraffin, and so on.

Examples of a commercially available product of the dimethylpolysiloxane include SH200 Series (such as SH200C Fluid 1CS, SH200C Fluid 2CS, SH200C Fluid 5CS, SH200C Fluid 10CS, SH200C Fluid 20CS, SH200C Fluid 30CS, SH200C Fluid 50CS, SH200C Fluid 100CS, SH200C Fluid 200CS, SH200C Fluid 350CS, SH200C Fluid 500CS, SH200C Fluid 1,000CS, SH200C Fluid 5,000CS, SH200 Fluid 1.5CS, SH200 Fluid 3,000CS, SH200 Fluid 10,000CS, SH200 Fluid 12,500CS, SH200 Fluid 30,000CS, SH200 Fluid 60,000CS, SH200 Fluid 100,000CS, and SH200 Fluid 1,000,000CS), BY11-026, BY22-020, BY22-029, BY22-050A, and BY22-060 (all of which are manufactured by Dow Corning Toray Co., Ltd.); TSF-451 Series (manufactured by Momentive Performance Materials Inc.); KF-96 Series, KF9008, and KM904 (all of which are manufactured by Shin-Etsu Chemical Co., Ltd.).

In the case of using the component (B)', from the viewpoint of reduction in entanglement during hair rinsing, easiness of brushing after towel drying, and quick drying properties in the hair detergent, the content or blending amount of the component (B)' in the detergent is preferably 0.01% by mass or more, more preferably 0.03% by mass or more, still more preferably 0.05% by mass or more, and yet still more preferably 0.1% by mass or more, and from the viewpoint of lathering during washing and easiness of blending, it is preferably 10% by mass or less, more preferably 7% by mass or less, still more preferably 5% by mass or less, yet still more preferably 3% by mass or less, even yet still more preferably 2% by mass or less, and even still more preferably 1.5% by mass or less.

A specific range of the content or blending amount of the component (B)' in the detergent is preferably 0.01 to 10% by mass, more preferably 0.03 to 7% by mass, still more preferably 0.05 to 5% by mass, yet still more preferably 0.1 to 3% by mass, even yet still more preferably 0.1 to 2% by mass, and even still more preferably 0.1 to 1.5% by mass.

In the case of using the component (B)' in the detergent of the present invention, from the viewpoint of lathering during washing and reduction in entanglement during hair rinsing, easiness of brushing after towel drying, quick drying properties, and easiness of blending in the hair detergent, a mass ratio [(A)/(B)'] of the component (A) to the component (B)' is preferably 1 or more, more preferably 3 or more, still more preferably 5 or more, and yet still more preferably 10 or more, and it is preferably 500 or less, more preferably 200 or less, still more preferably 100 or less, and yet still more preferably 50 or less.

A specific range of the mass ratio [(A)/(B)'] of the component (A) to the component (B)' is preferably 1 to 500, more preferably 3 to 200, still more preferably 5 to 100, and yet still more preferably 5 to 50.

<Component (D): Cationic Polymer>

For the purpose of lathering during washing and strengthening reduction in entanglement during hair rinsing, easiness of brushing after towel drying, and quick drying properties in the hair detergent, the detergent of the present invention may be one further containing or being prepared by further blending, as a component (D), a cationic polymer.

In the present invention, the cationic polymer refers to a water-soluble polymer having a cationic group and having a cationic charge as a whole, and the cationic group refers to a cation group or a group capable of being ionized to become a cation group.

Examples of the cationic polymer that is used as the component (D) include a cationized polygalactomannan, such as cationized guar gum, cationized tara gum, and cationized locust bean gum; a cationized cellulose; a cationized hydroxyalkyl cellulose, such as cationized hydroxyethyl cellulose and cationized hydroxypropyl cellulose; a cationic starch; a cationized polyvinyl alcohol; a quaternized dialkylaminoalkyl (meth)acrylic acid salt polymer, such as a vinylpyrrolidone/N,N-dimethylaminoethyl methacrylic acid diethyl sulfate copolymer and an N,N-dimethylaminoethyl methacrylic acid diethyl sulfate/N,N-dimethylacrylamide/dimethacrylic acid polyethylene glycol copolymer; a diallyl quaternized ammonium salt polymer, such as a polydiallyldimethylammonium chloride, a diallyldimethylammonium chloride/acrylic acid copolymer, a diallyldimethylammonium chloride/acrylamide copolymer, and a diallyldimethylammonium chloride/acrylic acid/acrylamide copolymer; a vinylimidazolium trichloride/vinylpyrrolidone copolymer; a vinylpyrrolidone/alkylamino (meth)acrylate copolymer; a vinylpyrrolidone/alkylamino (meth)acrylate/vinyl caprolactam copolymer; a vinylpyrrolidone/(meth)acrylamide propyl trimethylammonium chloride copolymer; an alkyl acrylamide/(meth)acrylate/alkylaminoalkyl acrylamide/polyethylene glycol (meth)acrylate copolymer; an adipic acid/dimethylaminohydroxypropyl ethylene triamine copolymer; and cationic polymers described in JP S53-139734 A and JP S60-36407 A. These may be used alone or in combination of two or more thereof. Of these, at least one selected from the group consisting of a cationized polygalactomannan, a cationized hydroxyalkyl cellulose, a quaternized dialkylaminoalkyl (meth)acrylic acid salt polymer, and a diallyl quaternized ammonium salt polymer is preferred; and at least one selected from the group consisting of cationized guar gum, a cationized hydroxyalkyl cellulose, and a quaternized dialkylaminoalkyl (meth)acrylic acid salt polymer is preferred.

From the viewpoint of lathering during washing and strengthening reduction in entanglement during hair rinsing, easiness of brushing after towel drying, and quick drying properties in the hair detergent, the weight average molecular weight of the component (D) is preferably 10,000 or more, more preferably 50,000 or more, and still more preferably 100,000 or more, and it is typically 8,000,000 or less.

Here, the weight average molecular weight may be, for example, measured by means of gel permeation chromatography (GPC) under the following condition.
Mobile layer: 50 mM LiBr, (1% $CH_3COOH$/ethanol)/water = 3/7
Column: TSK gel $\alpha$-M (two columns connected in series)
Standard substance: Polyethylene glycol

As a commercially available cationic polymer that may be used as the component (D), for example, the following may be exemplified.

(Cationized guar gum)

JAGUAR Excel (manufactured by Solvay (Novecare)), JAGUAR C-14-S (manufactured by Solvay-Rhodia), etc.

(Cationized tara gum)

CATINAL CTR-100 (manufactured by Toho Chemical Industry Co., Ltd.), etc.

(Cationized locust bean gum)

CATINAL CLB-100 (manufactured by Toho Chemical Industry Co., Ltd.), etc.

(Cationized hydroxyethyl cellulose)

Polyquaternium-10 (o-[2-hydroxy-3-(trimethylammonio)propyl]hydroxyethyl cellulose chloride): for example, UCare Polymer JR-400 (manufactured by The Dow Chemical Company), POIZ C-60H (manufactured by Kao Corporation), POIZ C-150L (manufactured by Kao Corporation), etc.

(Cationized hydroxypropyl cellulose)

SOFCARE C-HP2W (manufactured by Kao Corporation), etc.

(Cationized polyvinyl alcohol)

GOHSENX K-434 (manufactured by The Nippon Synthetic Chemical Industry Co., Ltd.), CM318 (manufactured by Kuraray Co., Ltd.), etc.

(Vinylpyrrolidone/N,N-dimethylaminoethyl methacrylic acid diethyl sulfate copolymer)

Polyquaternium-11: GAFQUAT 734 (manufactured by ISP Japan Ltd.), GAFQUAT 755N (manufactured by ISP Japan Ltd.), etc.

(N,N-Dimethylaminoethyl methacrylic acid diethyl sulfate/N,N-dimethylacrylamide/dimethacrylic acid polyethylene glycol copolymer)

Polyquaternium-52: SOFCARE KG-101W-E (manufactured by Kao Corporation), etc.

(Polydiallyldimethylammonium chloride)

Polyquaternium-6: MERQUAT 100 (manufactured by Lubrizol Advanced Materials, Inc.), etc.

(Diallyldimethylammonium chloride/acrylic acid copolymer)

Polyquaternium-22: MERQUAT 280 and MERQUAT 295 (all of which are manufactured by Lubrizol Advanced Materials, Inc.), etc.

(Diallyldimethylammonium chloride/acrylamide copolymer)

Polyquaternium-7: MERQUAT 550 (manufactured by Lubrizol Advanced Materials, Inc.), etc.

(Diallyldimethylammonium chloride/acrylic acid/acrylamide copolymer)

Polyquaternium-39: MERQUAT 3331PR (manufactured by Lubrizol Advanced Materials, Inc.), etc.
In the case of using the component (D), from the viewpoint of lathering during washing and strengthening reduction in entanglement during hair rinsing, easiness of brushing after towel drying, and quick drying properties in the hair detergent, the content or blending amount of the component (D) in the detergent is preferably 0.01% by mass or more, more preferably 0.05% by mass or more, still more preferably 0.1% by mass or more, and yet still more preferably 0.3% by mass or more, and from the viewpoint of lathering during washing and strengthening reduction in entanglement during hair rinsing, easiness of brushing after towel drying, and quick drying properties in the hair detergent and the viewpoint of easiness of blending, it is preferably 5% by mass or less, more preferably 3% by mass or less, still more preferably 2% by mass or less, and yet still more preferably 1% by mass or less.
A specific range of the content or blending amount of the component (D) in the detergent is preferably 0.01 to 5% by mass, more preferably 0.05 to 3% by mass, still more preferably 0.1 to 2% by mass, and yet still more preferably 0.3 to 1% by mass.
In the case of using the component (D), from the viewpoint of lathering during washing and strengthening reduction in entanglement during hair rinsing, easiness of brushing after towel drying, and quick drying properties in the hair detergent and the viewpoint of easiness of blending, a mass ratio [(A)/(D)] of the component (A) to the component (D) in the detergent is preferably 0.1 or more, more preferably 0.5 or more, still more preferably 1 or more, yet still more preferably 3 or more, even yet still more preferably 5 or more, and even still more preferably 10 or more, and it is preferably 100 or less, more preferably 50 or less, still more preferably 30 or less, and yet still more preferably 20 or less.
A specific range of the mass ratio [(A)/(D)] of the component (A) to the component (D) in the detergent is preferably 0.1 to 100, more preferably 0.5 to 50, still more preferably 1 to 30, yet still more preferably 3 to 20, even yet still more preferably 5 to 20, and even still more preferably 10 to 20.

<Component (E): Ampholytic Surfactant>

For the purpose of lathering during washing and strengthening reduction in entanglement during hair rinsing, easiness of brushing after towel drying, and quick drying properties in the hair detergent, the detergent of the present invention may be one further containing or being prepared by further blending, as a component (E), an ampholytic surfactant.
Examples of the ampholytic surfactant include betaine-based surfactants, such as an imidazoline-based betaine, an alkyldimethylamino acetic acid betaine, and a fatty acid amidopropyl betaine; and sultaine-based surfactants, such as an alkyl sultaine, an alkyl hydroxysultaine, an alkylamidosultaine, and an alkylamidohydroxy sultaine. These may be used alone or in combination of two or more thereof.
As the betaine-type surfactant, a fatty acid amidopropyl betaine is preferred. The fatty acid amidopropyl betaine is preferably one having an acyl group having 8 or more and 22 or less carbon atoms, and furthermore, 10 or more and 18 or less carbon atoms; more preferably at least one selected from the group consisting of lauric acid amidopropyl betaine (lauramidopropyl betaine), palm kernelamidopropyl betaine, and cocamidopropyl betaine; and still more preferably lauric acid amidopropyl betaine.
The sultaine-based surfactant is preferably an alkyl hydroxysultaine; more preferably an alkyl hydroxysultaine having an alkyl group having 8 or more and 22 or less carbon atoms; and furthermore, 10 or more and 18 or less carbon atoms; and still more preferably lauryl hydroxysultaine.
From the viewpoint of lathering during washing and strengthening reduction in entanglement during hair rinsing, easiness of brushing after towel drying, and quick drying properties in the hair detergent, as the component (E), it is preferred to use a betaine-based surfactant and a sultaine-based surfactant in combination; it is more preferred to use a fatty acid amidopropyl betaine and an alkyl hydroxysultaine in combination; and it is still more preferred to use lauric acid amidopropyl betaine and lauryl hydroxysultaine in combination.
In the case of using the component (E), from the viewpoint of lathering during washing and reduction in entanglement during hair rinsing and easiness of brushing after towel drying in the hair detergent, the content or blending amount of the component (E) in the detergent is preferably 0.01% by mass or more, more preferably 0.1% by mass or more, still more preferably 0.3% by mass or more, and yet still more preferably 0.5% by mass or more, and from the viewpoint of lathering during washing and reduction in entanglement during hair rinsing, easiness of brushing after towel drying, quick drying properties, and easiness of blending in the hair detergent, it is preferably 10% by mass or less, more preferably 7% by mass or less, still more preferably 5% by mass or less, and yet still more preferably 3% by mass or less.
A specific range of the content or blending amount of the component (E) in the detergent is preferably 0.01 to 10% by mass, more preferably 0.1 to 7% by mass, still more preferably 0.3 to 5% by mass, and yet still more preferably 0.5 to 3% by mass.
In the case of using the component (E), from the viewpoint of lathering during washing; and reduction in

entanglement during hair rinsing, easiness of brushing after towel drying, quick drying properties; and easiness of blending in the hair detergent, a mass ratio [(A)/(E)] of the component (A) to the component (E) in the detergent is preferably 0.1 or more, more preferably 0.5 or more, still more preferably 1 or more, and yet still more preferably 5 or more, and it is preferably 100 or less, more preferably 75 or less, still more preferably 50 or less, yet still more preferably 30 or less, and even yet still more preferably 20 or less.

A specific range of the mass ratio [(A)/(E)] of the component (A) to the component (E) in the detergent is preferably 0.1 to 100, more preferably 0.5 to 75, still more preferably 1 to 50, yet still more preferably 5 to 30, and even yet still more preferably 5 to 20.

<Component (F): Nonionic Surfactant>

For the purpose of lathering during washing and strengthening reduction in entanglement during hair rinsing in the hair detergent, the detergent of the present invention may be one further containing or being prepared by further blending, as a component (F), a nonionic surfactant.

Examples of the nonionic surfactant include a polyoxyalkylene alkyl ether, a polyoxyalkylene alkenyl ether, a polyoxyalkylene sorbitan fatty acid ester, a polyoxyalkylene fatty acid ester, an alkyl glucoside, an alkyl glyceryl ether, a higher fatty acid sugar ester, a polyglycerin fatty acid ester, a polyoxyethylene hydrogenated castor oil, an alkyl saccharide, an alkylamine oxide, and an alkylamidoamine oxide.

Of these, at least one selected from the group consisting of a polyoxyalkylene alkyl ether, a polyoxyalkylene alkenyl ether, a polyoxyalkylene sorbitan fatty acid ester, a polyoxyalkylene fatty acid ester, an alkyl glucoside, and an alkyl glyceryl ether is preferred; and at least one selected from the group consisting of a polyoxypropylene alkyl ether and an alkyl glyceryl ether is more preferred.

The carbon number of each of the alkyl group in the polyoxyalkylene alkyl ether, the alkyl glycoside, the alkyl glyceryl ether, the alkyl saccharide, the alkylamine oxide, and the alkylamidoamine oxide; the alkenyl group in the polyoxyalkylene alkenyl ether; and the fatty acid in the polyoxyalkylene sorbitan fatty acid ester, the polyoxyalkylene fatty acid ester, the higher fatty acid sugar ester, and the polyglycerin fatty acid ester is preferably 8 or more and 22 or less, more preferably 8 or more and 18 or less, and still more preferably 8 or more and 12 or less.

In the case of using the component (F), from the viewpoint of lathering during washing, the content or blending amount of the component (F) in the detergent is preferably 0.01% by mass or more, more preferably 0.05% by mass or more, still more preferably 0.1% by mass or more, and even yet still more preferably 0.3% by mass or more, and from the same viewpoint as above, it is preferably 10% by mass or less, more preferably 5% by mass or less, still more preferably 3% by mass or less, and yet still more preferably 2% by mass or less.

A specific range of the content or blending amount of the component (F) in the detergent is preferably 0.01 to 10% by mass, more preferably 0.05 to 5% by mass, still more preferably 0.1 to 3% by mass, and yet still more preferably 0.3 to 2% by mass.

In the case of using the component (F), from the viewpoint of lathering during washing and reduction in entanglement during hair rinsing, easiness of brushing after towel drying, quick drying properties, and easiness of blending in the hair detergent, a mass ratio [(A)/(F)] of the component (A) to the compound (F) in the detergent is preferably 0.1 or more, more preferably 0.5 or more, still more preferably 1 or more, and yet still more preferably 5 or more, and it is preferably 100 or less, more preferably 75 or less, still more preferably 50 or less, and yet still more preferably 30 or less.

A specific range of the mass ratio [(A)/(F)] of the component (A) to the compound (F) in the detergent is preferably 0.1 to 100, more preferably 0.5 to 75, still more preferably 1 to 50, and yet still more preferably 5 to 30.

<Aqueous Medium>

The detergent of the present invention is one typically formed by containing or blending an aqueous medium. Examples of the aqueous medium include water; lower alcohols, such as ethanol and isopropanol; and low-molecular diols and triols having 6 or less carbon atoms, such as 1,3-butylene glycol, glycerin, ethylene glycol, and propylene glycol, with water being preferred. Although the content or blending amount of the aqueous medium in the detergent may be appropriately selected according to the dosage form of the detergent, it is typically in a range of 5 to 99% by mass, and preferably in a range of 30 to 98% by mass.

<Other Component>

The detergent of the present invention may appropriately contain or be prepared by blending therein the other component within a range where the object of the present invention is not impaired. Examples of the other component include compounds that are typically blended in a hair detergent, such as an antioxidant, an oil agent, an anti-dandruff agent, a vitamin agent, a disinfectant, an anti-inflammatory agent, an antiseptic, a chelating agent, a moisturizer, a

pearlescent agent, a ceramide, a fragrance, and a UV absorber.

<pH>

From the viewpoint of lathering during washing, a pH of the detergent of the present invention is preferably 3.0 or higher, more preferably 3.2 or higher, and still more preferably 4.3 or higher in terms of a pH of a 5% aqueous dispersion, and from the viewpoint of inhibition of entanglement of hair or the like, it is preferably 7.0 or lower, more preferably 6.5 or lower, still more preferably 6.0 or lower, and yet still more preferably 5.6 or lower in terms of a pH of a 5% aqueous dispersion. A specific range of the pH of the 5% aqueous solution of the detergent of the present invention is preferably 3.0 to 7.0, more preferably 3.2 to 6.5, still more preferably 4.3 to 6.0, and yet still more preferably 4.3 to 5.6.
The aforementioned pH is a measured value at 25°C, and specifically, it may be measured by the method described in the section of Examples.
A production method of the detergent of the present invention is not particularly limited. For examples, the detergent of the present invention may be produced by blending the components (A) to (C) and other components to be used, if desired by the method described in the section of Examples and mixing the contents by using a known agitation apparatus or the like.
A use method of the detergent of the present invention is not particularly limited. The entanglement of hair or fibers during washing and after washing can be prevented through a step of washing a washing object, such as hair and fibers, by a known method by using the detergent of the present invention.
Regarding the aforementioned embodiments, the present invention discloses a detergent and a method of preventing entanglement of hair or fibers.

<1> A detergent containing or prepared by blending:

(A) at least one anionic surfactant selected from the group consisting of alkyl sulfuric acid salts, alkyl ether sulfuric acid salts, and alkyl ether carboxylic acid salts;
(B) an aminopolyether-modified silicone satisfying the following formula (I) and containing a polyoxyalkylene structure having a repeating unit represented by the general formula (2):

$$0.5 \le \mathrm{Si/AO} \le 1.2 \qquad \mathrm{(I)}$$

(in the formula (I), Si represents a molar number of a silicon atom in the component (B), and AO represents an oxyalkylene average addition molar number in the component (B)), and

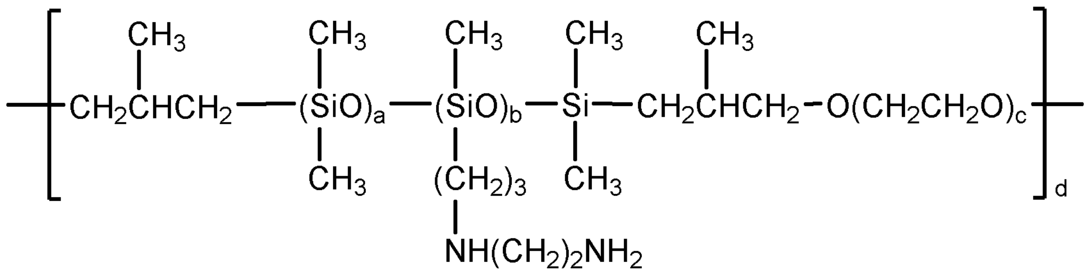
(2)

(in the formula (2), a represents a number of 2 or more and 100 or less; b represents a number of 1 or more and 50 or less; c represents a number of 10 or more and 18 or less; d represents a number of 1 or more and 100 or less; and the bonding order among the structural units within the bracket does not matter, and the bonding form may be a block form or a random form); and
(C) at least one organic acid selected from the group consisting of lactic acid, succinic acid, citric acid, tartaric acid, and malic acid,
wherein,
a kinematic viscosity at 25°C of the component (B) is 500 to 1,500 $mm^2/s$,
a mass ratio [(A)/(B)] of the component (A) to the component (B) is 5 to 20, and
a mass ratio [(A)/(C)] of the component (A) to the component (C) is 30 to 100.

<2> The detergent as set forth in <1>, wherein the Si/AO in the formula (I) is 0.7 or more and 0.9 or less.

<3> The detergent as set forth in <1> or <2>, wherein the kinematic viscosity at 25°C of the component (B) is 700 to 1,400 $mm^2/s$.
<4> The detergent as set forth in any one of <1> to <3>, wherein the content or blending amount of the component (A) is 5 to 18% by mass.
<5> The detergent as set forth in any one of <1> to <4>, wherein the content or blending amount of the component (B) is 0.1 to 3% by mass.
<6> The detergent as set forth in any one of <1> to <5>, which is a hair detergent.
<7> A method for preventing entanglement of hair or fibers, the method including a step of washing hair or fibers by using the detergent as set forth in any one of <1> to <6>.

Examples

The present invention is hereunder described by reference to Examples, but it should be construed that the present invention is not limited to the scope of the Examples. In the present Examples, various measurements and evaluations were performed by the following methods.

(pH Measurement)

The pH at 25°C was measured using a pH meter (F-51, manufactured by Horiba, Ltd.). For the pH measurement of a hair detergent, an aqueous dispersion having a hair detergent diluted with purified water to 5% was used.

(Measurement of Si/AO in Component (B) and Component (B1)')

10 mg of each of the component (B) and the component (B1)' (aminopolyether-modified silicones 1 to 3) used in each of the Examples was weighed in a No. 3 screw vial and dried overnight in a nitrogen atmosphere. The obtained dried material was added with and dissolved in 1 mL of deuterated chloroform (manufactured by Wako Pure Chemical Industries, Ltd., Chloroform-d, 99.8%). Using $^1$H-NMR (manufactured by Bruker Japan K.K., JH074504 AV400), a unit ratio of Si/AO was calculated from an integrated value of the dimethylsilyl group bonded to silicon (6H: -0.3 to 0.3 ppm) and an integrated value of the oxyethylene (EO) chain (4H: 3.3 to 3.9 ppm) according to the following equation.

$$(\text{Si/AO}) = [(\text{Integrated value at -0.3 to 0.3 ppm})/6]/[(\text{Integrated value at 3.3 to 3.9 ppm})/4]$$

(Lathering Evaluation)

A hair bundle of untreated hairs of a Chinese person having a mass of 20 g and a length of 20 cm was damaged through a bleach treatment with the following hair bleach and washed with the following plain shampoo, to obtain a hair bundle for lathering evaluation. This hair bundle for evaluation was thoroughly damped with warm water at 35 to 40°C and then coated with 1 g of a hair detergent of each of the Examples, followed by washing for 1 minute.
The lathering evaluation was performed by five panelists, and by scoring the lowest as "1" and the highest as "5", respectively, a 5-grade evaluation was performed while defining Comparative Example 1 as a standard score 3. An average grade (rounded off to the first decimal place) of the evaluation by the five panelists was tabulated. When the average grade by the five panelists is score 4 or above, it may be said that an explicitly excellent performance is revealed in that evaluation.

(Evaluation Criteria)

5: Lathering is very good.
4: Lathering is good.
3: Lathering is moderate.
2: Lathering is bad.
1: Lathering is very bad.

(Composition of Plain Shampoo)

| Component | (% by mass) |
|---|---|
| Polyoxyethylene lauryl ether sulfuric acid Na | 11.3 (*1) |
| Coconut fatty acid N-methylethanolamide (*2) | 3.0 |
| Citric acid | 0.2 |
| Methyl paraben | 0.3 |
| Purified water | Balance |
| total | 100.0 |

*1: 42.0% by mass as EMAL E-27C (manufactured by Kao Corporation, active ingredient: 27% by mass)
*2: AMION C-11S (manufactured by Kao Corporation)

(Composition of Hair Bleach)

| Component | (% by mass) |
|---|---|
| Monoethanolamine | 1.5 |
| 28% by mass ammonia water | 4.0 |
| Ammonium bicarbonate | 1.0 |
| 35% by mass hydrogen peroxide water | 8.2 |
| Purified water | Balance |
| total | 100.0 |

(Bleach Treatment Method)

A hair bundle of untreated hairs of a Chinese person having a mass of 20 g was coated with 20 g of the hair bleach, allowed to stand for 30 minutes, and then thoroughly rinsed with warm water at 35 to 40°C. This operation was repeated four times, thereby preparing a bleach-treated hair bundle.

(Measurement of Combing Load)

The aforementioned hair bundle for lathering evaluation was prepared, thoroughly damped with warm water at 35 to 40°C, and then coated with 1 g of a hair detergent of each of the Examples. The resulting hair bundle was lightly put between palms of both hands and washed for 1 minute while moving the hands so as to rub together. Thereafter, the hair bundle was rinsed with warm water for 30 seconds without allowing the fingers to pass therethrough, thereby obtaining a hair bundle for measurement of combing load.
The aforementioned hair bundle was set on a combing force measuring apparatus (manufactured by Utsunomiyaseiki Co., Ltd., "KOT-0303"), a comb (one in which ten pins having a diameter of 2 mm were linearly arranged at intervals of 4 mm) was allowed to pass through the uppermost part of the hair bundle, and a load (gf) applied when passing the comb through the hair ends was measured. The load was calculated at 200 points of the hair bundle having a length of 20 cm, and a total value of the 200 points in total was designated as "combing load".

(Easiness of Brushing after Towel Drying)

The aforementioned hair bundle for lathering evaluation was prepared, thoroughly damped with warm water at 35 to 40°C, and then coated with 1 g of a hair detergent of each of the Examples. The resulting hair bundle was lightly put between palms of both hands and washed for 1 minute while moving the hands so as to rub together. Thereafter, the hair bundle was rinsed with warm water for 30 seconds without allowing the fingers to pass therethrough. The obtained hair bundle was placed on a towel, and the both surfaces of the hair bundle were covered by the towel and subjected to towel drying so as to rub together, thereby obtaining a hair bundle for brushing evaluation.
The aforementioned hair bundle was set on a combing force measuring apparatus (manufactured by Utsunomiyaseiki Co., Ltd., "KOT-0303"), the root of the hair bundle was put from both sides between two brushes, and an action of simultaneously stroking the brushes towards the hair end was repeated 10 times. A maximum load during one stroke was measured, and the number of strokes required until the load became not more than 400 gf was evaluated. It is meant that as the number of strokes shown in the table is small, after towel drying, the entanglement of hair is small, too, and the brushing is readily performed.

(Evaluation of Quick Drying Properties)

The aforementioned hair bundle for brushing evaluation was prepared, dried by blowing hot air with a hair dryer (manufactured by Can Co., Ltd., "Sobis TYPE 315", air volume setting: High) at a distance of 5 to 15 cm from the hair bundle while combing fingers, and a time until the wet hair returned to the weight in a dry state was measured. It is meant that as the drying time is short, the quick drying properties are high.

Examples 1 to 8 and Comparative Examples 1 to 4 (Preparation and Evaluation of Hair Detergent)

A hair detergent of each of the Examples was prepared in the following way according to the blending shown in each table and evaluated.

With respect to Examples 1 to 5 and Comparative Examples 1 to 4, the components (A) and (C) were uniformly dissolved in an appropriate amount of water, the components (B) and/or (B)' were/was added, and the contents were uniformly mixed.

With respect to Examples 6 to 8, the component (D) was dissolved or uniformly dispersed in water, an appropriate amount of water and the components (A), (C), (E), and (F) and other component(s) were added, and the contents were warmed to 80°C and uniformly mixed, followed by cooling to 40°C. The components (B) and (B)' were added thereto and uniformly mixed, and the moisture vaporized upon warming was finally replenished.

Using this hair detergent, the lathering evaluation and the measurement of combing load were carried out by the aforementioned methods.

In addition, with respect to Examples 2, 3, and 6 to 8 and Comparative Example 1, the evaluation of easiness of brushing after towel drying was carried out by the aforementioned method. With respect to Examples 3 and 6 to 8 and Comparative Example 1, the evaluation of quick drying properties was carried out by the aforementioned method. The results are shown in Tables 1 and 2.

The blending amounts shown in the tables are the active ingredient amount (% by mass) of each of the components.

Table 1

| | | | | Example 1 | Example 2 | Example 3 | Example 4 | Example 5 | Comparative Example 1 | Comparative Example 2 | Comparative Example 3 | Comparative Example 4 |
|---|---|---|---|---|---|---|---|---|---|---|---|---|
| Blending (% by mass) | (A) | Ammonium laureth sulfate | *1 | 10.0 | 10.0 | 10.0 | 10.0 | 10.0 | 10.0 | 10.0 | 10.0 | 10.0 |
| | (B) | Aminopolyether-modified silicone 1 (Si/AO = 0.80) | *2 | 0.5 | 0.5 | 0.5 | 1.0 | 1.0 | 1.0 | 4.0 | | |
| | (C) | Malic acid | *3 | 0.05 | 0.1 | 0.3 | 0.1 | 0.2 | 3 | 0.5 | 0.3 | 0.3 |
| | (B)' | Aminopolyether-modified silicone 2 (Si/AO = 2.46) | *4 | | | | | | | | 0.5 | |
| | | Aminopolyether-modified silicone 3 (Si/AO = 2.39) | *5 | | | | | | | | | 0.5 |
| | Others | Purified water | | | | | | | | | | |
| Mass ratio (A)/(B) | | | | 20 | 20 | 20 | 10 | 10 | 10 | 2.5 | - | - |
| Mass ratio (A)/(C) | | | | 200 | 100 | 33 | 100 | 50 | 3 | 20 | 33 | 33 |
| Evaluation | Lathering | | | 5 | 5 | 4 | 5 | 5 | 3 | 1 | 2 | 2 |
| | Combing load (gf) | | | 63825 | 60729 | 29441 | 54905 | 51130 | 97194 | 90424 | 140518 | 173273 |
| | Easiness of brushing after towel drying (the number of strokes required until the maximum load per stroke became not more than 400 gf) | | | - | 2 | 1 | - | - | 3 | - | - | - |
| | Quick drying properties (drying time/sec) | | | - | - | 137 | - | - | 179 | - | - | - |

*1: Manufactured by Kao Corporation, EMAL 170S-A (active ingredient: 70%)
*2: Manufactured by Dow Corning Toray Co., Ltd., SILSTYLE 201
*3: Manufactured by Wako Pure Chemical Industries, Ltd., DL-malic acid
*4: Manufactured by Dow Corning Toray Co., Ltd., SILSTYLE 104
*5: Manufactured by Dow Corning Toray Co., Ltd., SS-3588

Table 2

| | | | | Example | | |
|---|---|---|---|---|---|---|
| | | | | 6 | 7 | 8 |
| Blending (% by mass) | (A) | Ammonium laureth sulfate | *1 | 11 | 9 | 15 |
| | | Sodium laureth acetate | *2 | 0.7 | 1.2 | 0.1 |
| | (B) | Aminopolyether-modified silicone 1 (Si/AO = 0.80) | * 3 | 1.0 | 0.5 | 2.0 |
| | (C) | Succinic acid | *4 | 0.07 | 0.07 | 0.07 |
| | | Malic acid | *5 | 0.10 | | 0.10 |
| | | Citric acid | *6 | 0.02 | 0.10 | 0.10 |
| | | Lactic acid | *7 | | 0.10 | |
| | (D) | Polyquaternium -10 | *8 | 0.1 | 0.2 | 0.4 |
| | | Guar hydroxypropyltrimonium chloride | *9 | 0.5 | 0.3 | 0.1 |
| | | Polyquaternium -52 | *10 | 0.01 | 0.1 | 0.2 |
| | | PPG-2 hydroxypropyltrimonium cellulose | *11 | 0.2 | 0.01 | 0.1 |
| | (E) | Lauramidopropyl betaine | *12 | 0.6 | 0.2 | 1.2 |
| | | Lauryl hydroxysultaine | *13 | 0.8 | 1.2 | 0.2 |
| | (F) | Isodecyl glyceryl ether | *14 | 0.2 | 0.5 | 0.8 |
| | | PPG-3 caprylyl ether | *15 | 0.8 | 0.5 | 0.2 |
| | (B)' | Aminopolyether-modified silicone 2 (Si/AO = 2.46) | *16 | 0.1 | | |
| | | Aminopolyether-modified silicone 3 (Si/AO = 2.39) | *17 | | 0.5 | 0.02 |
| | | Dimethicone | *18 | 0.2 | 0.7 | 1.2 |
| | Others | Ethylene glycol distearate | *19 | 1.2 | 1.2 | 1.2 |
| | | Antiseptic | | Moderate amount | | |
| | | pH modifier | | Amount at which the pH of the 5% aqueous dispersion becomes 5% | | |
| | | Water | | Balance | | |
| Mass ratio (A)/(B) | | | | 12 | 20 | 8 |
| Mass ratio (A)/(C) | | | | 61 | 37 | 55 |
| Mass ratio (A)/(D) | | | | 14 | 17 | 19 |
| Mass ratio (A)/(E) | | | | 8 | 7 | 11 |
| Mass ratio (A)/(F) | | | | 12 | 10 | 15 |
| Mass ratio (A)/(B)' | | | | 39 | 9 | 12 |

(continued)

| | | Example | | |
|---|---|---|---|---|
| | | 6 | 7 | 8 |
| Evaluation | Lathering | 5 | 5 | 5 |
| | Combing load (gf) | 37371 | 43514 | 11679 |
| | Easiness of brushing after towel drying (the number of strokes required until the maximum load per stroke became not more than 400 gf) | 0 | 0 | 0 |
| | Quick drying properties (drying time/sec) | 96 | 116 | 91 |

*1: Manufactured by Kao Corporation, EMAL 170S-A (active ingredient: 70%)
*2: Manufactured by Kao Corporation, KAO AKYPO LM-26SD (active ingredient: 19%)
*3: Manufactured by Dow Corning Toray Co., Ltd., SILSTYLE 201
*4: Manufactured by Wako Pure Chemical Industries, Ltd., succinic acid
*5: Manufactured by Wako Pure Chemical Industries, Ltd., DL-malic acid
*6: Manufactured by Wako Pure Chemical Industries, Ltd., citric acid
*7: Manufactured by Wako Pure Chemical Industries, Ltd., lactic acid
*8: Manufactured by Kao Corporation, POIZ C-150L
*9: Manufactured by Solvay-Rhodia, JAGUAR C-14-S
*10: Manufactured by Kao Corporation, SOFCARE KG-101W-E (active ingredient: 2.4%)
*11: Manufactured by Kao Corporation, SOFCARE C-HP2W
*12: Manufactured by Kao Corporation, AMPHITOL 20AB
*13: Manufactured by Kao Corporation, AMPHITOL 20HD
*14: Manufactured by Kao Corporation, PENETOL GE-ID
*15: Manufactured by Kao Corporation, KAO SOFCARE GP-1
*16: Manufactured by Dow Corning Toray Co., Ltd., SILSTYLE 104
*17: Manufactured by Dow Corning Toray Co., Ltd., SS-3588
*18: Manufactured by Dow Corning Toray Co., Ltd., BY22-029 (active ingredient: 50%)
*19: Manufactured by Kao Corporation, EMANON 3201M-V

Industrial Applicability

In accordance with the detergent of the present invention, lathering during washing is favorable, and the generation of entanglement of a washing object, such as hair and fibers, even during washing and after washing can be substantially inhibited and solved without performing an operation of putting fingers through hair or the like. For example, in the case where the detergent of the present invention is a hair detergent, the entanglement in hair after washing and towel drying is hardly generated, and therefore, the hair can be dried for a short time, and finish after drying becomes favorable.

## Claims

**1.** A detergent comprising:

(A) an anionic surfactant,
(B) an aminopolyether-modified silicone containing a polyoxyalkylene structure and satisfying the following formula (I):

$$0.01 \leq \mathrm{Si/AO} \leq 1.6 \qquad \text{(I)}$$

wherein Si represents a molar number of a silicon atom in the component (B), and AO represents an oxyalkylene average addition molar number in the component (B), and
(C) an organic acid,
wherein a mass ratio [(A)/(B)] of the component (A) to the component (B) is less than 25, and

a mass ratio [(A)/(C)] of the component (A) to the component (C) is 30 or more and 3,000 or less.

**2.** A detergent, which is prepared by blending:

(A) an anionic surfactant,
(B) an aminopolyether-modified silicone containing a polyoxyalkylene structure and satisfying the following formula (I):

$$0.01 \leq \mathrm{Si/AO} \leq 1.6 \qquad \text{(I)}$$

wherein Si represents a molar number of a silicon atom in the component (B), and AO represents an oxyalkylene average addition molar number in the component (B), and
(C) an organic acid,
wherein a mass ratio [(A)/(B)] of the component (A) to the component (B) is less than 25, and
a mass ratio [(A)/(C)] of the component (A) to the component (C) is 30 or more and 3,000 or less.

**3.** The detergent according to claim 1 or 2, wherein the component (B) has a repeating unit represented by the following general formula (1):

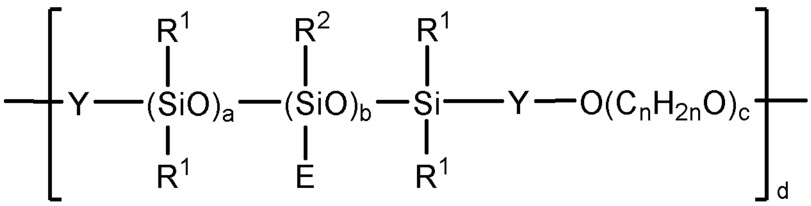
(1)

wherein $R^1$ represents a hydrogen atom or a monovalent hydrocarbon group having 1 or more and 6 or less carbon atoms; $R^2$ represents either $R^1$ or E; E represents a monovalent group represented by $-R^3-Z$, wherein $R^3$ represents a single bond or a divalent hydrocarbon group having 1 or more and 20 or less carbon atoms, and Z represents a primary to tertiary amino group-containing group; Y represents an alkylene group having 1 or more and 6 or less carbon atoms; a represents a number of 2 or more; b represents a number of 1 or more; c represents a number of 4 or more and 100 or less; and d represents a number of 1 or more; n represents a number of 2 or more and 10 or less; the bonding order among the structural units within the bracket does not matter, and the bonding form may be a block form or a random form; the $C_nH_{2n}O$'s in the number of c may be the same as or different from each other; and plural $R^1$'s, $R^2$'s, and E's may be the same as or different from each other, respectively.

**4.** The detergent according to any one of claims 1 to 3, wherein the component (C) is at least one selected from the group consisting of a monovalent carboxylic acid having a hydroxy group and a polyvalent carboxylic acid which may have a hydroxy group.

**5.** The detergent according to any one of claims 1 to 4, which further comprises or is prepared by further blending, as a component (B)', a silicone other than the component (B).

**6.** The detergent according to claim 5, wherein the component (B)' is at least one selected from the group consisting of an aminopolyether-modified silicone other than the component (B), an amino-modified silicone, a polyether-modified silicone, a dimethylpolysiloxane, a methylphenylpolysiloxane, a fatty acid-modified silicone, an alkoxy-modified silicone, and an alkyl-modified silicone.

**7.** The detergent according to any one of claims 1 to 6, which further contains or is prepared by further blending, as a component (D), a cationic polymer.

**8.** The detergent according to any one of claims 1 to 7, which further comprises or is prepared by further blending, as a component (E), an ampholytic surfactant.

**9.** The detergent according to any one of claims 1 to 8, which further comprises or is prepared by further blending, as

a component (F), a nonionic surfactant.

**10.** The detergent according to any one of claims 1 to 9, wherein the content or blending amount of the component (A) is 1% by mass or more and 30% by mass or less.

**11.** The detergent according to any one of claims 1 to 10, which is a hair detergent.

## INTERNATIONAL SEARCH REPORT

International application No.
PCT/JP2019/022962

A. CLASSIFICATION OF SUBJECT MATTER
Int.Cl. A61K8/898(2006.01)i, A61K8/362(2006.01)i, A61K8/365(2006.01)i, A61K8/39(2006.01)i, A61K8/46(2006.01)i, A61Q5/02(2006.01)i, C11D1/37(2006.01)i, C11D1/83(2006.01)i, C11D1/94(2006.01)i, C11D3/20(2006.01)i, C11D3/37(2006.01)i
According to International Patent Classification (IPC) or to both national classification and IPC

B. FIELDS SEARCHED
Minimum documentation searched (classification system followed by classification symbols)
Int.Cl. A61K8/00-8/99, A61Q1/00-90/00, C11D1/00-19/00, D06M13/00-15/715

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | JP 2012-207019 A (SHISEIDO CO., LTD.) 25 October 2012, paragraphs [0006]-[0008], [0014], [0095] & WO 2012/124766 A1 & TW 201249473 A | 1-11 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 August 2019 (07.08.2019) | 20 August 2019 (20.08.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 9151119 A
- JP 2007161597 A
- JP S53139734 A
- JP S6036407 A